(19) Europäisches Patentamt
European Patent Office
Office européen des brevets

(11) Publication number: **0 434 020 B1**

(12) **EUROPEAN PATENT SPECIFICATION**

(45) Date of publication of patent specification: **28.06.95**

(51) Int. Cl.[6]: **C07J 71/00**, A61K 31/58

(21) Application number: **90124739.5**

(22) Date of filing: **19.12.90**

# (54) 2,19-Methyleneoxy and 2,19-methylenethio bridged steroids as aromatase and 19-hydroxylase inhibitors.

(30) Priority: **20.12.89 US 453441**

(43) Date of publication of application:
**26.06.91 Bulletin 91/26**

(45) Publication of the grant of the patent:
**28.06.95 Bulletin 95/26**

(84) Designated Contracting States:
**AT BE CH DE DK ES FR GB GR IT LI LU NL SE**

(56) References cited:
**US-A- 3 627 756**
**US-A- 3 954 980**
**US-A- 4 814 324**

(73) Proprietor: **MERRELL DOW PHARMACEUTICALS INC.**
**2110 East Galbraith Road**
**Cincinnati**
**Ohio 45215 (US)**

(72) Inventor: **Peet, Norton P.**
**8028 Chestershire Drive**
**Cincinnati,**
**Ohio 45241 (US)**
Inventor: **Johnston J.O'Neil**
**9 Crooked Creek/Foxwood**
**Milford,**
**Ohio 45150 (US)**
Inventor: **Burkhart, Joseph P.**
**7290 Barrett Road**
**West Chester,**
**Ohio 45069 (US)**

(74) Representative: **VOSSIUS & PARTNER**
**Postfach 86 07 67**
**D-81634 München (DE)**

EP 0 434 020 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The estrogen hormones, estrone and estradiol, are involved in many physiological processes. The formation of these steroids is regulated by a number of enzymes. The enzyme aromatase is the rate limiting enzyme in the non-reversible conversion of the androgen hormones, testosterone and androstenedione, to the estrogen hormones, estradiol and estrone. Compounds such as aromatase inhibitors may thus regulate or inhibit androgen to estrogen conversion, and have therapeutic utility in treating clinical conditions potentiated by the presence of estrogens.

19-Nordeoxycorticosterone (19-norDOC) is known to induce mineralocorticoid hypertension. In the biosynthetic formation of 19-norsteroids, such as 19-norDOC, the initial step is the adrenal hydroxylation of an appropriate steroid such as deoxycorticosterone (DOC). The inhibition of the biosynthetic formation of 19-norDOC by inhibition of 19-hydroxylation of DOC would thus serve to decrease the level of 19-norDOC present in the animal involved and reduce hypertensive effects attributable to the presence of this material.

In US-A-3954980 a method of contraception is described, whereby steroidal 11,19-hemiacetals of 11-hydroxy-19-oxo compounds are dispensed orally to a human subject.

US-A-4814324 discloses sterol inhibitors of testosterone 5α-reductase produced by the controlled aerobic fermentation of a fungus of the genus Gliocladium, ATCC No 20826. They are useful in the treatment and prevention of acne, seborrhea, femal hirsutism and benign prostatic hypertrophy.

N-dialkylaminoalkyl-N-(2β,19-epoxy-5α-androstan-17β-yl) amines/formamides and 3α-halo derivatives thereof are described in US-A-3627756. These compounds exhibit valuable pharmacological properties, e.g. antiulcerogenic, antimicrobial and anti-inflammatory.

The present invention is directed to 2,19-bridged steroidal aromatase and 19-hydroxylase inhibitor compounds, their related intermediates, and the process for their preparation. These compounds may be represented by the following formula (I)

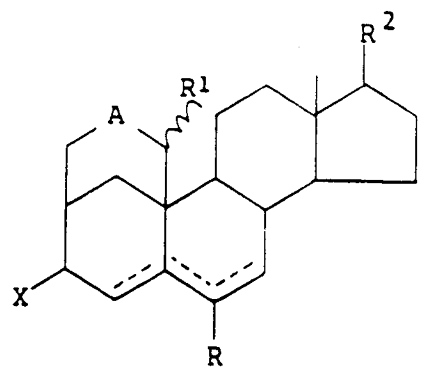

(I)

wherein

------ represents a single or double bond,

A is O, S, SO, or $SO_2$,

R is H, $=CH_2$, =O, or -OH,

$R^1$ is H or $C_{1-4}$ alkyl,

$R^2$ is =O, -OH, or -O-($C_{1-4}$ alkanoyl),

X is =O, $=CH_2$, -OH, -O-($C_{1-4}$ alkanoyl), or -CO-$CH_2$-Y,

y is H, -OH, or -O($C_{1-4}$ alkanoyl), and when $R^2$ is -CO-$CH_2$Y, X may not include -OH, and R may not include =O or -OH.

The compounds of the present invention are inhibitors of aromatase and 19-hydroxylase. As aromatase inhibitors, they are useful in treating hyperestrogenemia. The compounds are useful in controlling abnormally high levels of estrogens, both when the high levels observed are relatively steady, or when there are brief surges of elevated levels occuring as part of cyclical body functions. Both females and males can be treated, although obviously, the level of estrogens which would be considered high in males would be much lower than the amount considered high in females. These compounds are also useful as anti-fertility agents to prevent ovulation or implantation in females, or to reduce the mating behavior in males where brain aromatization is required for such behavior. These compounds further have value in treating gynecomastia, male infertility resulting from elevated estrogen levels, and hyperestrogenemia, which may proceed myocardial infarction. The compounds also may be used to treat breast cancer and other various estrogen-induced or estrogen-stimulated tumors and hyperplastic tissue disorders.

The bioconversion of deoxycorticosterone via a 19-hydroxylase pathway to 19-nordeoxycorticosterone potentiates its mineralcorticoid activity. Mineralcorticoid excess results in a syndrome characterized by hypokalemia, metabolic alkalosis, polydipsia, polyuria, and hypertensive conditions. Increased excretion of 19-nordeoxycorticosterone has been reported for hypertensive patients, including those with primary aldosteronism, Cushing's syndrome, 17α-hydroxylase deficiency, and individuals with essential hypertension. As 19-hydroxylase inhibitors, these compounds may be useful as antihypertensive agents and for management of edemous conditions often associated with sodium retention and potassium loss.

To achieve their desired effect, the compounds of the present invention may be administered orally, parenterally, for example, intravenously, intraperitoneally, intramuscularly, or subcutaneously, including the injection of the active ingredient directly into tissue or tumor sites, to a patient in need of treatment. The term patient is taken to mean a warm-blooded animal, for example, mammals such as humans, primates, cattle, dogs, cats, horses, sheep, mice, rats, and pigs. These compounds may also be administered in the form of a pharmaceutical preparation, and may further be incorporated into sustained delivery devices. The amount of compound administered will vary over a wide range and be any effective amount. Depending on the patient to be treated, the condition to be treated, and mode of administration, the effective amount of compound administered will vary from about 0.01 to 150 mg/kg of body weight per day, and preferably from about 0.1 to 50 mg/kg body weight per day.

For oral administration, the compounds can be formulated into solid or liquid preparations, such as capsules, pills, tablets, troches, powders, solutions, suspensions, or emulsions. The solid unit dosage forms can be a capsule which can be of the ordinary gelatin type containing the active compound and a carrier, for example, lubricants and inert filler such a lactose, sucrose and corn starch. In another embodiment, an active compound of the invention can be tableted with conventional tablet bases such as lactose, sucrose and corn starch in combination with binders such as acacia, corn starch, or gelatin, disintegrating agents such as potato starch, or alginic acids and a lubricant such as stearic acid or magnesium stearate.

For parenteral administration the compounds may be administered as injectable dosages of a solution or suspension of the compound in a physiological acceptable diluent with a pharmaceutical carrier which can be a sterile liquid such as water-in-oil with or without the addition of a surfactant and other pharmaceutically acceptable adjuvants. Illustrative of oils which can be employed in these preparations are those of petroleum, animal, vegetable or synthetic origin, for example, peanut oil, soybean oil, and mineral oil. In general, water, saline, aqueous dextrose and related sugar solutions, ethanols and glycols, such as propylene glycol or polyethylene glycol are preferred liquid carriers, particularly for injectable solutions.

The compounds can be administered in the form of a cutaneous patch, a depot injection, or implant preparation which can be formulated in such a manner as to permit a sustained release of the active ingredient. The active ingredient can be compressed into pellets or small cylinders and implanted subcutaneously or intramuscularly as depot injections or implants. Implants may employ inert materials such as biodegradable polymers and synthetic silicones, for example, Silastic®, silicone rubber manufactured by Dow Corning Corporation. Further information on suitable pharmaceutical carriers and formulation techniques are found in standard texts such as Remington's Pharmaceutical Sciences, Mack Publishing Company, Easton, Pennsylvania.

Inhibition of aromatase activity is demonstrated by using laboratory methods similar to procedures described in U.S. Patent No. 4,322,416, and as published in Johnston et al., Endocrinology 115:776, 1984, and Burkhart et al., Steroids 45:357, 1985.

In this assay, the inhibitor is preincubated with enzyme prior to assaying for activity in the presence of high substrate levels. A time-related decrease in enzyme activity can be indicative of irreversible binding of the inhibitor with the enzyme.

In the time-dependent assay, an amount of the enzyme inhibitor in 100 µl of the assay buffer described above which will provide assay concentrations which are usually between 1 nM and 10 µM are added to 35 ml centrifuge tubes containing 600 µl of the NADPH generating system. The preincubation is started by the addition of 700 µl of aromatase preparation, usually 500-800 µg of microsomal protein per ml of assay buffer. These preparations are mixed using a vortex mixer and incubated for 0, 10, 20, or 40 minutes at 25°C. Then 100 µl of androstenedione (~6.8 µM) containing 1β-$^3$H androstenedione is added in assay buffer to provide an assay concentration of substrate (0.55 µM) which is at least ten times the $K_m$ of androstenedione (0.04 µM). Following vortexing, the enzyme incubation is continued for 10 minutes before being terminated by the addition of chloroform. The amount of radioactivity in the aqueous fraction is determined by scintillation procedures. The enzymatic activity for each concentration of inhibitor at each time period of preincubation is calculated as a percent of the "0" minute vehicle control arbitrarily set at 100%. Therefore, the present enzyme inhibition is expressed as a percentage: (100 percent minus percent

enzyme activity with inhibitor present).

Enzyme kinetic analysis utilized Kitz-Wilson plots for time-dependent assays. These analyses provide estimates of apparent $K_i$ of inactivation which represents the inhibitor concentration required to produce half-maximal rate of enzyme inactivation. The pseudo first-order rate constant for enzyme inactivation ($k_{cat}$) and the half-time of inactivation ($\tau_{50}$) of infinite inhibitor concentrations were determined. The ratio of $k_{cat}/K_i$ (inactivation) provides an index number which increases with increased efficiency of enzyme inactivation and increased inhibitor affinity for the enzyme active site. The compound below (4), [3R-(3α, 6aα,6bα,8aβ,11aα,11bβ)]-3,4,6b,7,8,8a,10,11,11a,11b,12,13-dodecahydro-8a-methyl-6H-3,6a-methanocyclopenta[5,6] napth[1,2-c]oxocin-2,9-dione, exhibited the following results:

$K_i$ (nM) = 17.6
$\tau_{50}$ (min) = 2.86
$k_{cat}/K_i$ = 227,300

When assaying compounds for 19-hydroxylase inhibiting activity, compounds were solubilized in dimethyl sulfoxide (DMSO) at 10 mM and diluted in DMSO to provide 0.01-10 μM final concentration when 2 μl aliquots were added to microcentrifuge assay tubes. Assay buffer (10 mM KCl, 1 mM EDTA, 100 mM Tris-HCl at pH 8.0) which had been supplemented with an NADPH-generating system to provide assay concentrations of 1 mM NADPH, 3 mM glucose-6-phosphate and 1 I.U./ml glucose-6-phosphate dehydrogenase were incubated at 37°C for 5 minutes prior to addition of hamster adrenal mitochondrial protein. Aliquots (180 μl) of this latter preparation containing 5.1 μg enzyme protein were assayed at 37°C for 5 minutes following the initiation of the assay by the addition of 20 μl of assay buffer containing radiolabeled DOC (0.85 μM final concentration, 0.01 μCi with 99.8% radiochemical purity, NEN Research Products, Boston, MA). Assays were quenched by the addition of 800 μl of 20% $CH_3CN$-2% HOAc. The reactants were centrifuged for 2 minutes at 15,000xg and analyzed by liquid chromatography (Beckman Instruments Inc., San Ramon, CA) on two $C_{18}$ Radial Pak columns (Waters, Millipore Corporation, Milford, MA) in series (5 μM particles, 0.8 × 10 cm each). Chromatographic buffer A was 10% $CH_3CN$-0.1% HOAc and buffer B was 80% $CH_3CN$-0.1% HOAc. The column was eluted at a flow rate of 1 ml/minute with a linear gradient from 0 to 30% buffer B over 36 minutes followed by 100% buffer B. The amount of remaining labeled DOC substrate and initial hydroxylated products, corticosterone and 19-hydroxy-DOC, were separated and the radioactivity contained in each peak quantitated. The 19-hydroxylase activity was based on the quantity of radiolabeled DOC metabolized, since corticosterone and 19-hydroxy-DOC are the products of a single enzyme.

Unlabeled steroids were monitered by their absorbance at 240 nm with a Kratus Spectroflow 773 detector (Kratus Analytical Instruments, Ramsey, NJ). The extinction coefficients for derivatives of DOC were asumed to be similar to that of DOC ($\epsilon$ = 17,200 $M^{-1}cm^{-1}$). Radioactivity of DOC metabolites was measured using an online Flow-One scintillation spectrometer (Radiomatic Instrument & Chemical Co., Inc., Tampa, FL) with a 1 ml flow cell.

Time-dependent enzyme inhibition was evaluated by preincubating the enzyme with steroidal compound for either 0 or 60 minutes at 37°C prior to the addition of radiolabeled substrate for a 5 minute assay. Apparent $K_m$ for the first hydroxylation of DOC may be estimated by the double reciprocal plot of Lineweaver-Burk. $IC_{50}$'s may be graphically estimated from linear-log plots of enzyme activities and log of inhibitor concentrations.

Various procedures may be used to prepare the compounds of the present invention. Scheme 1 is utilized to prepare compound (4) below, [3R-(3α,6aα,6bα,8aβ,11aα,11bβ)]-3,4,6b,7,8,8a,10,11,11a,11b,12,13-dodecahydro-8a-methyl-6H-3,6a-methanocyclopenta[5,6]napth[1,2-c]oxocin-2,9-dione. Alternatively, compound (4) may be named 2,19-(methyleneoxy) androst-4-ene-3,17-dione. To facilitate the understanding of the present invention, steroidal nomenclature and numbering are utilized in the procedures and examples that follow.

## SCHEME 1

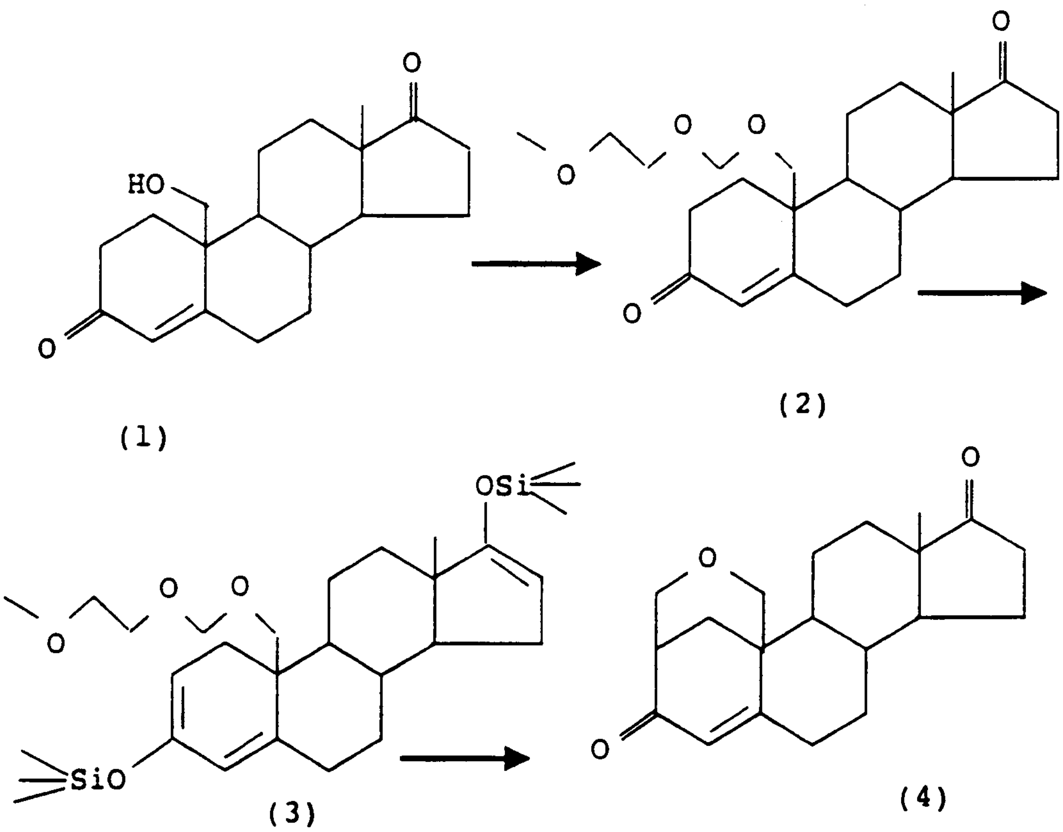

(1) (2) (3) (4)

Commercially available steroidal starting compound (1) is reacted with diisopropylethylamine and 1-chloro-2,5-dioxahexane to form the compound, 19-[(2-methoxyethoxy) methoxy]-androst-4-ene-3,17-dione (2). This compound is then reacted with a mixture of trimethylsilychloride in diisopropylamine and n-BuLi to form the compound 19-[(2-methoxyethoxy)methoxy]-3,17-bis[(trimethylsilyl)oxy]androst-2,4,16-triene (3). This compound is then treated with $TiCl_4$ to give the desired compound, 2,19-(methyleneoxy)androst-4-ene-3,17-dione (4). Alternatively, to prepare those compounds wherein A = S, the corresponding 19-thio steroidal starting compound is utilized, and the reaction proceeds analogous to Scheme 1. The compounds where A = SO and A = $SO_2$ are prepared from the corresponding compound where A = S by treatment with one or two equivalents of 3-chloroperoxybenzoic acid, respectively, in a solvent such as methylene chloride.

To prepare the compound bearing the hydroxyacetyl substituent at the 17-position (10), Scheme 2 is utilized:

SCHEME 2

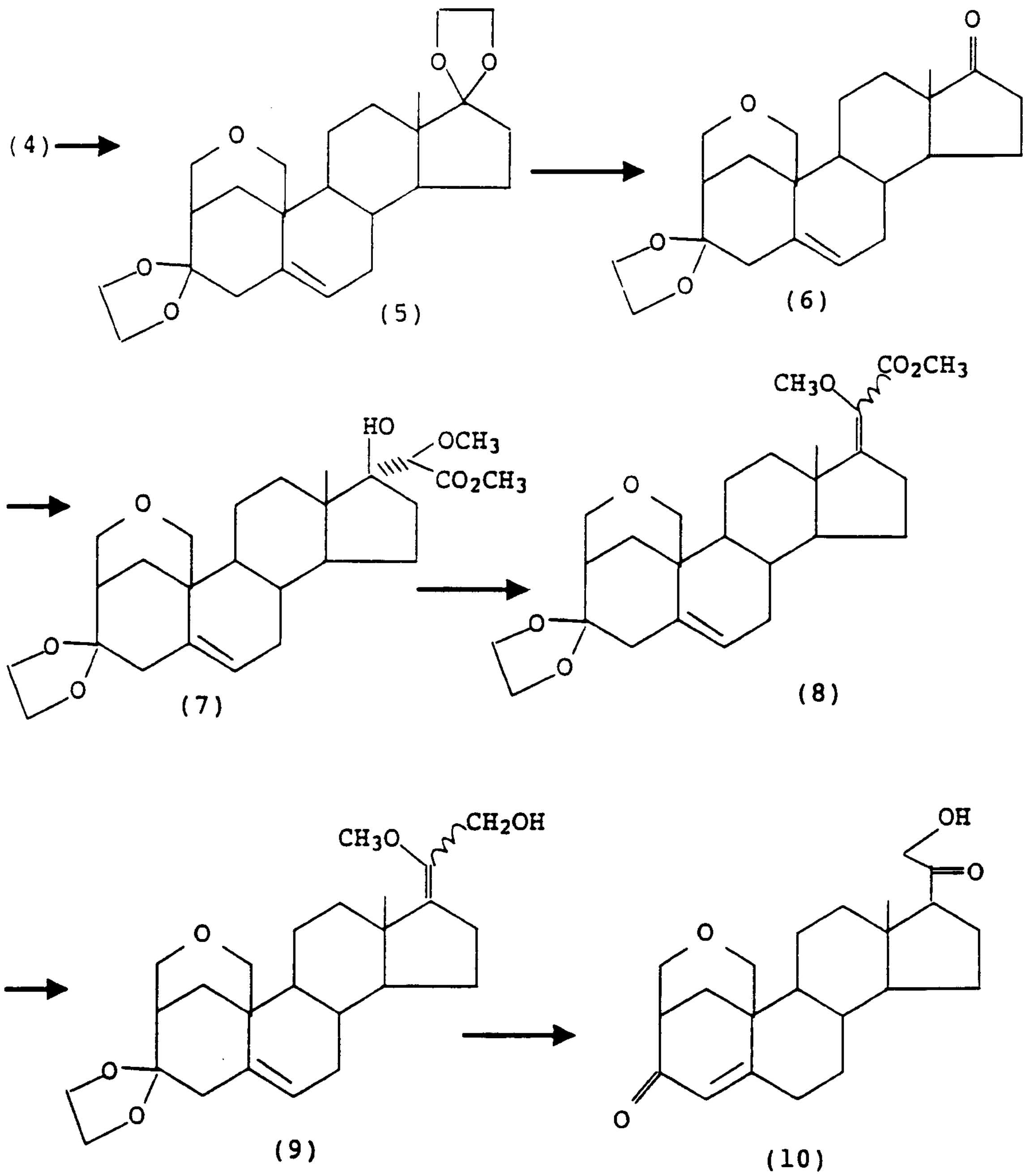

(4) (5) (6) (7) (8) (9) (10)

The 2,19-(methyleneoxy)androst-4-ene-3,17-dione (4) starting compound is treated with a catalytic amount of acid such as methanesulfonic acid in an excess of ethylene glycol to form the corresponding 3,17-bis(ethylenedioxy) compound (5). This compound is then selectively hydrolyzed at the 17-position with 0.15% aqueous perchloric acid in t-butanol and dichloromethane to give the corresponding 17-ketone (6). The ketone is then reacted with methyl methoxyacetate and lithium diisopropylamide whereupon the indicated ester (i.e., the methylene group thereof), adds across the 17-ketone to give the 17-substituted 17-hydroxy steroid (7). Dehydration introduces a 17-exocyclic double bond and the resulting methoxy ester (8) is reduced with a hydride reducing agent such as diisobutylaluminum hydride to give the corresponding alcohol (9), which is then further treated with acid to hydrolyze the enol ether and also the 3-ketal to give the desired 21-hydroxy-20-keto compound (10).

To prepare compound (14), 2,19-(methyleneoxy)androst-4-ene-3,6,17-trione, Scheme 3 is utilized:

**SCHEME 3**

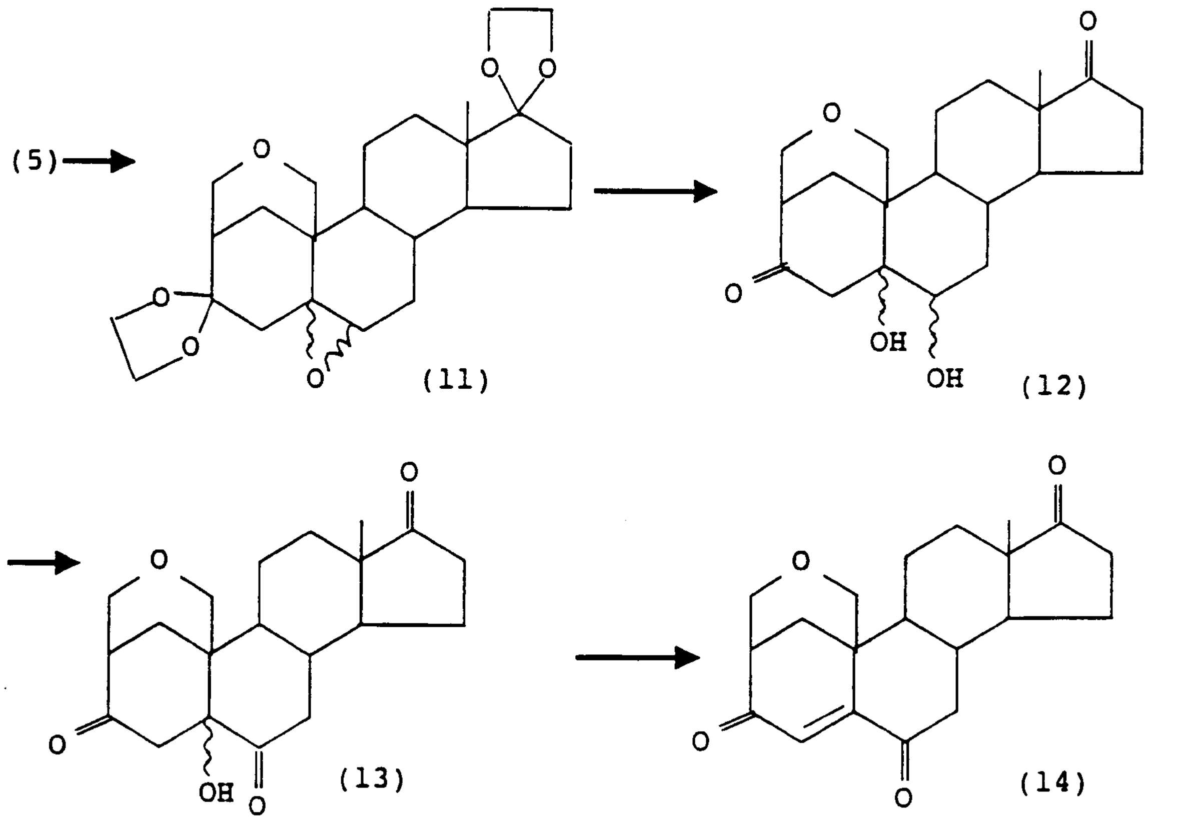

(5) (11) (12) (13) (14)

The diketal starting material (5) is treated with metachloroperbenzoic acid in dichloromethane at 0 ° C to produce the epoxide (11). The epoxide is opened to the corresponding diol (12) using perchloric acid in THF and $H_2O$. The ketals are also removed in this process. The diol is then oxidized to the hydroxy-ketone by Jones oxidation. The hydroxy-ketone (13) is then taken up in benzene and dehydrated using p-toluenesulfonic acid to yield the steroidal trione (14).

Compounds containing multiple double bonds on the steroid ring system can be obtained by dehydrogenation of the appropriate starting compound. For example, dehydrogenation of 2,19-(methyleneoxy)-androst-4-ene-3,17-dione (4) with chloranil in t-butanol gives the corresponding diene (15) as shown by Scheme 4 below.

**SCHEME 4**

(4)

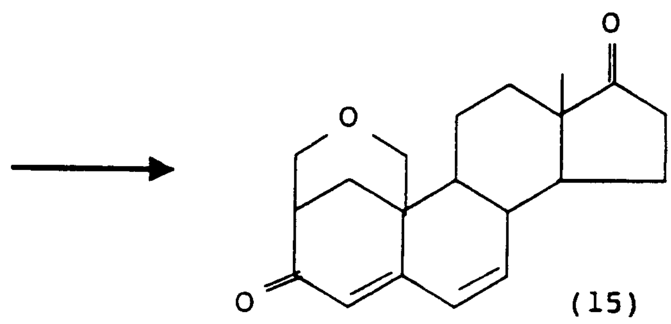

(15)

To obtain compounds of the present invention wherein R is $=CH_2$, 2,19-(methyleneoxy)androst-4-ene-3,17-dione (4) is reacted with a formaldehyde acetal as shown by Scheme 5 below.

## SCHEME 5

(4)

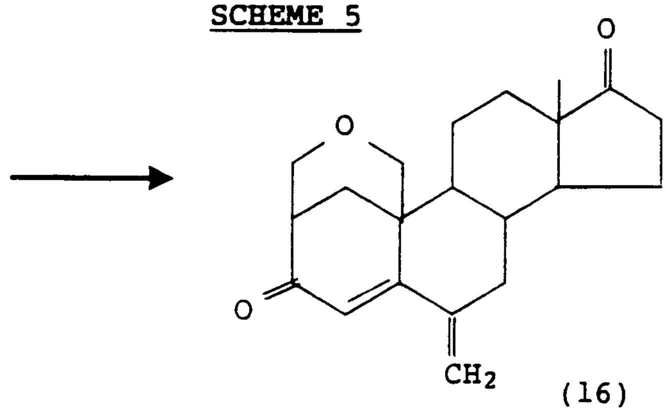

(16)

Reagents such as p-toluenesulfonic acid, strong mineral acids, acidic ion exchange resin, or preferentially, phosphoryl chloride with formaldehyde dimethyl or diethyl acetal, are most suitable to effect this condensation.

To obtain 2,19-(methyleneoxy)androst-4-ene-3,17-diol (19), Scheme 6 is utilized:

## SCHEME 6

(4)

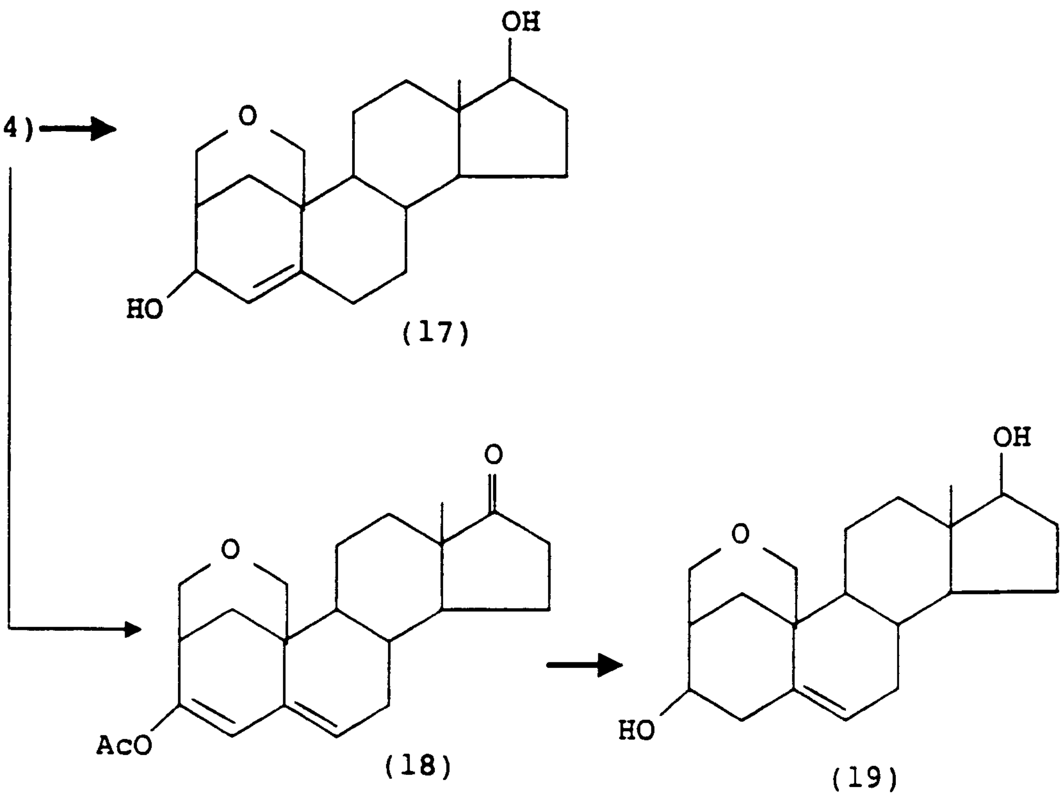

(17)

(18)

(19)

The starting compound, 2,19-(methyleneoxy)androst-4-ene-3,17-dione (4), is reduced with sodium borohydride in ethanol to yield the corresponding diol (17). To prepare the 5,6-ene diol (19), the starting compound, 2,19-(methyleneoxy)androst-4-ene-3,17-dione (4), is treated with a catalytic amount of p-toluenesulfonic acid and heating in a solvent such as $Ac_2O$. The mixture is then cooled. To this mixture is then added pyridine followed by ethanol to yield the dienol acetate (18).

Alternatively, the dienol acetate (18) may preferably be prepared by adding an excess of $Ac_2O$ and a catalytic amount of 70% aqueous $HClO_4$ to the steroid (4) in EtOAc. The mixture is then stirred for 15

minutes and poured into dilute $Na_2CO_3$, extracted, and washed with dilute $Na_2CO_3$ and brine to yield the dienol acetate (18). The dienol acetate (18) is then treated with calcium borohydride in EtOH at -15 °C. The reaction is quenched with HOAc and partitioned between EtOAc and $H_2O$ to yield the diol (19). Treatment of the diol (19), with anhydride, such as acetic anhydride, gives the corresponding diacetate.

To prepare the compound wherein $R^1$ is $CH_3$, Scheme 7 is utilized.

**SCHEME 7**

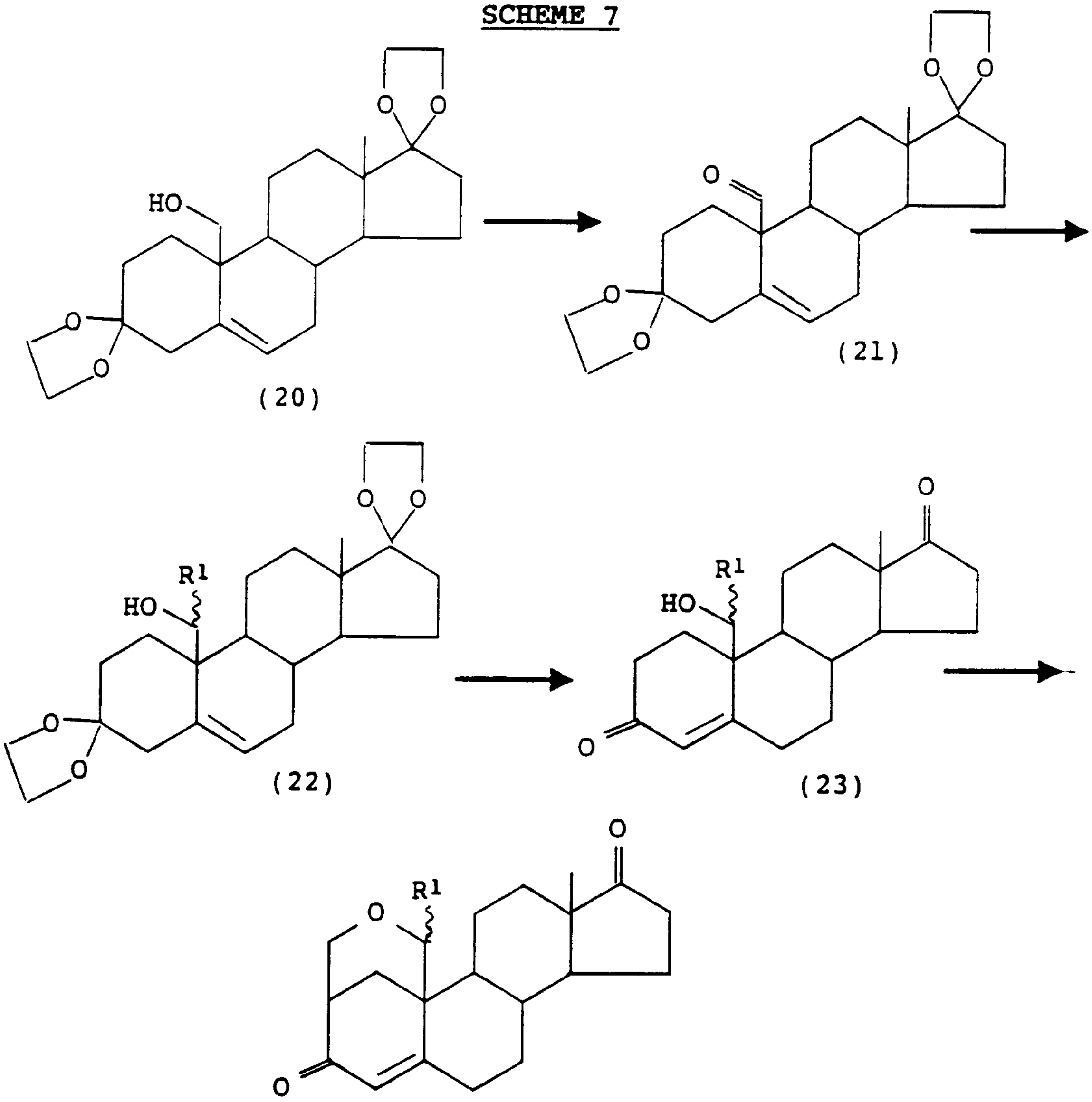

(20) (21) (22) (23) (24)

The known bisketal compound (20) undergoes a Swern oxidation to yield the oxidized bisketal (21). This compound is then treated with $R^1MgBr$ or $R^1Li$, wherein $R^1$ is defined above, to produce the $R^1$-substituted hydroxy compound (22). Treatment of (22) with aqueous HCl in THF yields the dione (23). Treatment of the dione (23) in a manner analogous to Scheme 1 yields the $R^1$-substituted 2,19-(methyleneoxy)androst-4-ene-3,17-dione (24).

To prepare the compound wherein X is $=CH_2$, Scheme 8 is utilized.

**SCHEME 8**

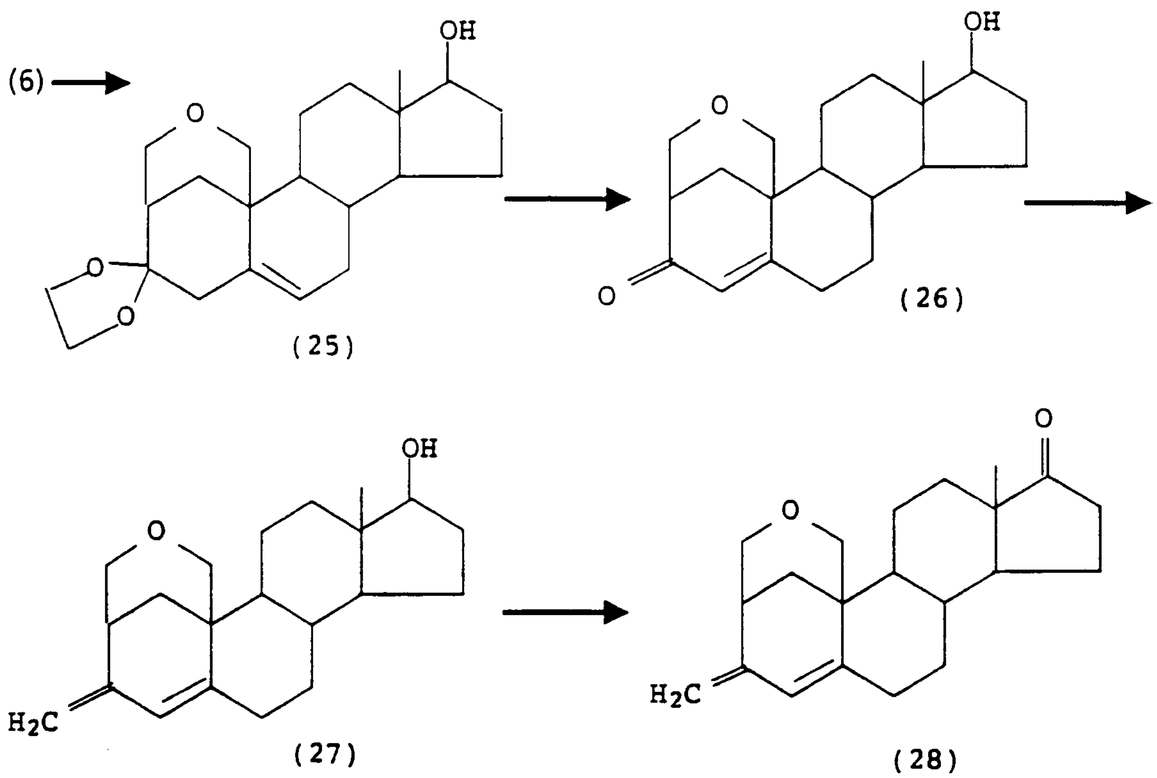

(6) (25) (26) (27) (28)

To the starting 17-keto compound (6), in EtOH, at 0 °C, is added an excess of $NaBH_4$. After 30 minutes, the reaction is quenched with $CH_3COCH_3$ and concentrated. The residue is added to $CH_2Cl_2$, washed with 0.5 N hydrochloric acid solution, water, then brine to yield the corresponding 17-hydroxy compound (25). To this compound (25) in THF is added aqueous hydrochloric acid solution to form the corresponding 3-keto-17-hydroxy compound (26). This compound (26) is then treated with $(C_6H_5)_3P=CH_2$ to yield the corresponding 3-methylene-17-hydroxy compound (27). This compound is then oxidized at C-17 by Jones oxidation to form the 3-methylene-17-keto compound (28). Optionally, compound (28) may then be treated in a manner analogous to Scheme 2 to form the corresponding 21-hydroxy-20-keto compound.

The following examples are provided to illustrate the present invention. They should not be construed as limiting it in any way.

## EXAMPLE 1

To a stirred solution of 19-hydroxyandrost-4-ene-3,17-dione (1) (4.54 g, 15.0 mmol)in $CH_2Cl_2$ (40 ml) under argon atmosphere was added di-isopropylethylamine (5.23 ml, 30.0 mmol) followed by 1-chloro-2,5-dioxahexane (2.57 ml, 22.5 mmol). After 20 hours, the reaction was diluted with $CH_2Cl_2$ (60 ml) and the organics were washed with $H_2O$ (75 ml), 0.5 N hydrochloric acid (2 x 75 ml), saturated $NaHCO_3$ (35 ml), and brine (75 ml). Drying ($MgSO_4$) and concentration gave an orange oil (6.33 g). The oil was dissolved in 10 ml of EtOAc/hexane (65:35) and loaded onto a column. Flash chromatography (7.5 x 15 cm silica gel column), eluting with EtOAc/hexane (65:35) gave 19-[(2-methoxyethoxy)methoxy]-androst-4-ene-3,17-dione (2). (Weight: 4.44 g). HRMS calculated for $C_{23}H_{34}O_5$ ($M^+$): 390.2406; found $M^+$: 390.2401; error = -1.3 ppm.

## EXAMPLE 2

To a stirred solution of diisopropylamine (0.37 ml, 2.65 mmol) in THF (7 ml) under argon and cooled to -20 °C was added n-BuLi (1.03 ml, 2.42 M in hexane, 2.49 mmol). After 12 minutes, a cooled (-20 °C)

solution of trimethylsilylchloride (0.74 ml, 5.81 mmol) in THF (1 ml) was added rapidly. After 2 minutes, a cooled (-20 °C) solution of the product of Example 1 (2) (324 mg, 0.83 mmol) in THF (2 ml) was added dropwise followed by a 0.5 ml THF rinse. The reaction was stirred at -20 °C for 30 minutes and then allowed to warm slowly to room temperature. The reaction was stirred at room temperature for 30 minutes, triethylamine (1 ml) was added and the reaction was diluted to a 50 ml volume with ethyl ether. The organics were washed with saturated $NaHCO_3$ (50 ml + 20 ml) followed by brine/saturated $NaHCO_3$ (20 ml of a 3:1 mixture). Drying ($MgSO_4$) and concentration gave a pale yellow oil. To this product was added hexane, the mixture concentrated, and then placed under high vacuum for 5 minutes to remove THF and triethylamine, yielding 19-[(2-methoxyethoxy)methoxy]-3,17-bis[(trimethylsilyl)oxy]androst-2,4,16-triene (3) (quantitative).

EXAMPLE 3

To a stirred solution of the product of Example 2 (3) (0.83 mmol) in $CH_2Cl_2$ (8 ml) under argon and cooled to -20 °C was rapidly added a $TiCl_4$ solution (2.49 ml of a lM $TiCl_4$ in $CN_2Cl_2$ solution, 2.49 mmol). A tan suspension resulted. Additional $CH_2Cl_2$ (8 ml) was added. The reaction suspension was stirred at -20 °C for 35 minutes, diluted with $CH_2Cl_2$ and poured into saturated $NaHCO_3$. The layers were separated and the aqueous layer was extracted with additional $CH_2Cl_2$. The combined organics were washed with saturated $NaHCO_3$ (2x), 0.5N hydrochloric acid (1x), followed by brine. Drying ($MgSO_4$) and concentration gave a milky oil. To this product was added 4 ml of EtOAc/hexane (50:50), the solid was crushed, and the suspension was heated with a heat gun and then allowed to cool to room temperature prior to loading the supernatant onto a flash column for chromatography (2 x 10 cm silica gel column). The supernatant was loaded as stated, eluted with EtOAc/ hexane (50:50), and 15-20 ml fractions where collected. Concentration of the product-containing fractions gave a pale yellow oil. $Et_2O$ was added to the residue and the flask was swirled to provide a solid. Concentration gave an oily, white solid (0.14 g). This product was then triturated with 2 ml of $Et_2O$/hexane (3:1). As much solid as possible was scraped from the side of the flask and the suspension was filtered to provide a white solid (56 mg). The solid was dried under high vacuum over refluxing acetone for 6 hours, yielding the compound of the formula below, mp 204-213 °C. [3R-(3α,6aα,6bα,8aβ, 11aα, 11bβ)]-3,4,6b,7,8,8a,10,11,11a,11b,12,13-dodecahydro-8a-methyl-6H-3,6a-methanocyclopenta[5,6]napth[1,2-c]oxocin-2,9-dione, or alternatively, 2,19-(methyleneoxy)androst-4-ene-3,17-dione. (Weight remained 56 mg.).

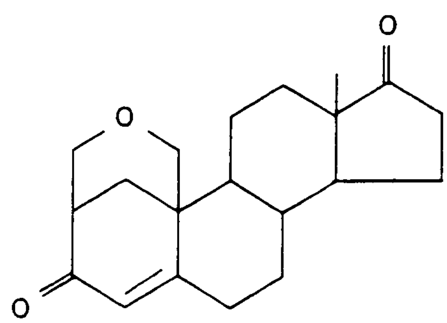

Elemental analysis: Calculated for $C_{20}H_{26}O_3$: C, 76.40; H, 8.34. Actual: C, 76.60; H, 8.53.

The corresponding sulfur compound, 2,19-(methylenethio) androst-4-ene-3,17-dione, was obtained in an analogous manner and melted at 183-199 °C.

EXAMPLE 4

The product of Example 3 is treated with a catalytic amount of methanesulfonic acid and an excess of ethylene glycol in solvent (benzene) and heated to reflux under Dean-Stark conditions to form the corresponding 3,17-bis[ethylenedioxy)-5-ene compound (5).

EXAMPLE 5

A solution of the product of Example 4 in dichloromethane and t-butanol is treated with 0.15% aqueous perchloric acid. The mixture is heated at gentle reflux for two hours with stirring and then allowed to cool to

room tempetature. The reaction mixture is then poured into saturated sodium carbonate solution and extracted into EtOAc. The EtOAc extract is washed with water and brine, dried over magnesium sulfate, concentrated, and chromatographed on silica gel eluting with EtOAc/hexane (2:3) to gig the corresponding 17-one compound (6).

EXAMPLE 6

A solution of methyl methoxyacetate in tetrahydrofuran is slowly added to a cold solution of lithium diisopropylamide, prepared from diisopropylamine and n-butyl lithium in hexane, in the same solvent. A solution of the product of Example 5 in tetrahydrofuran is then added dropwise over 5-10 minutes and the solution is stirred for three hours at the same temperature. Saturated aqueous ammonium chloride solution is then added dropwise, and the mixture is poured into ice water and extracted with ethyl acetate. The extract is washed with brine, dried over sodium sulfate, filtered, and concentrated to afford the 17-substituted steroid (7). The crude product is chromatographed on silica gel, eluting with 1:1 ethyl acetate:hexane to afford the product as a mixture of isomers.

EXAMPLE 7

A solution of the product of Example 6 in pyridine and $CH_2Cl_2$ is chilled to 0 °C and treated dropwise with thionyl chloride over 5-10 minutes. After stirring for 75 minutes at the same temperature, the solution is poured into ice water. The organic layer is washed twice with brine, dried over sodium sulfate, filtered and concentrated to afford crude product. Flash chromatography (30% ethyl acetate/70% hexane) affords the methoxyester (8).

EXAMPLE 8

A solution of the product of Example 7 in toluene is chilled to -20 °C and treated dropwise with a 20% solution of diisobutylaluminum hydride in hexane. The solution is stirred at -20 °C for 30 minutes. Water is added and the mixture is stirred at 0 °C for 20 minutes, poured into ice water and extracted with 3:1 ether:dichloromethane. The extracts are washed with brine, dried over sodium sulfate, and concentrated. The residue is subjected to flash chromatography eluting with 3:2 ethyl acetate:hexane to afford the alcohol (9).

EXAMPLE 9

To a solution of the product of Example 8 in THF is added 0.5 N aqueous HCl. After 4 days, the reaction is diluted with $CH_2Cl_2/H_2O$, the layers separated, and the organics washed with brine, dried over sodium sulfate,and concentrated. The hydroxyketone of the formula below is isolated by flash chromatography (silica gel) eluting with EtOAc/hexane (4:1).

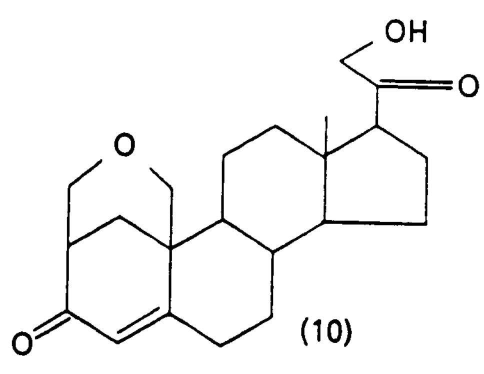

(10)

HRMS:

Calcd for $C_{22}H_{31}O_4$: $MH^+$ 359.2222

Found: $MH^+$ 359.2204

Error: 5.0 ppm

EXAMPLE 10

To a solution of the product of Example 4 (5) is added m-chloroperbenzoic acid in methylene chloride at 0 °C. The mixture is maintained at 0 °C for 16 hours then diluted with methylene chloride and washed with water, 10% sodium carbonate, and brine, then dried and evaporated. Chromatography gives the epoxide (11).

EXAMPLE 11

To a solution of the product of Example 10 (11) in THF and water is added dropwise 70% aqueous perchloric acid and the reaction is stirred at room temperature for 48 hours. The mixture is diluted with methylene chloride, washed with aqueous $Na_2CO_3$ and brine, then dried ($MgSO_4$) and concentrated. Chromatography gives the corresponding diol (12).

EXAMPLE 12

To the product of Example 11 (12) in acetone at 0 °C is added dropwise Jones' reagent until a brown color persists for 15 minutes. The reaction is quenched with methanol. The mixture is then partioned between methylene chloride and water. The organic phase is washed with brine, and then dried and concentrated. Chromatography gives the hydroxyketone (13).

EXAMPLE 13

To the product of Example 12 (13) dissolved in benzene is added a catalytic amount of p-toluenesulfonic acid. The mixture is heated at reflux for 30 minutes using a Dean-Stark water trap. The cooled solution is then poured into water. The organics are washed with aqueous $Na_2CO_3$ and brine, then dried and evaporated. The residue is chromatographed to afford the trione compound of the formula below, 2,19-(methyleneoxy)androst-4-ene-3,6,17-trione (14).

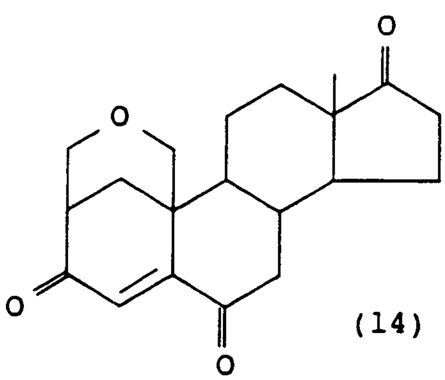

(14)

EXAMPLE 14

To the product of Example 3 (4) in t-butyl alcohol is added chloranil (1.2 equivalents). The mixture is refluxed for 3 hours, cooled, then concentrated. The residue is taken up in $CHCl_3$ and washed with water, aqueous NaOH, and brine. Drying and concentration, followed by chromatography affords the compound of the formula:

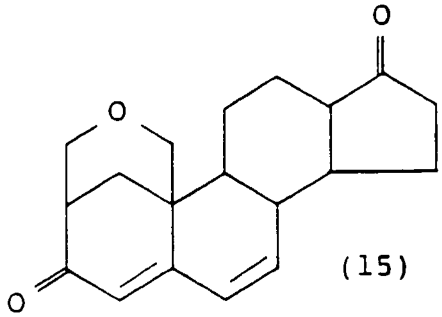

(15)

EXAMPLE 15

A suspension of sodium acetate in absolute chloroform containing formaldehyde dimethyl acetal and phosphoryl chloride is stirred at reflux for 1 hour. After addition of the product of Example 3 (4), the mixture is treated dropwise with phosphoryl chloride over a period of 2.5 hours. The reaction is subsequently stirred at reflux for the appropriate time. The suspension is allowed to cool and under vigorous stirring a saturated aqueous solution of sodium carbonate is added dropwise until the pH of the aqueous layer becomes alkaline. The organic layer is separated, washed with water, and dried with sodium sulfate. After concentration and purification, the product obtained is the compound of the formula:

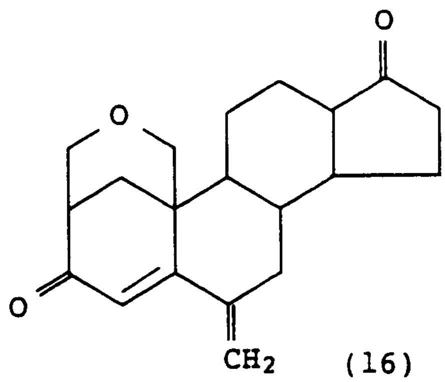

(16)

## Claims

**Claims for the following Contracting States : AT, BE, CH, DE, DK, FR, GB, IT, LI, LU, NL, SE**

**1.** A compound of the formula (I)

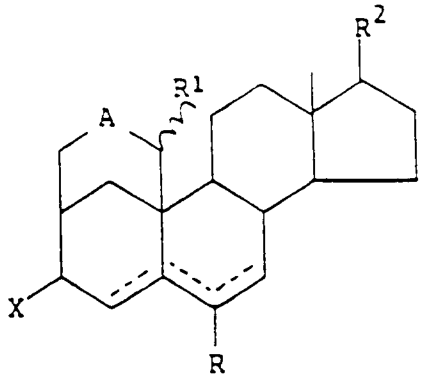

(I)

wherein
------- represents a single or double bond,
A is 0, S, SO, or $SO_2$,
R is H, $=CH_2$, $=O$, or -OH,
$R^1$ is H or $C_{1-4}$ alkyl,
$R^2$ is $=O$, -OH, -O-($C_{1-4}$ alkanoyl), or -CO-$CH_2$Y,
X is $=O$, $=CH_2$, -OH, or -O-($C_{1-4}$ alkanoyl), and
Y is H, -OH, or -O-($C_{1-4}$ alkanoyl), and when $R^2$ is -CO-$CH_2$Y, X may not include -OH, and R may not include $=O$ or -OH.

2. The compound (I) of Claim 1, wherein $R^2$ is $=O$, -OH, or -O-($C_{1-4}$ alkanoyl).

3. The compound of Claim 1, wherein $R^2$ is -CO-$CH_2$Y and Y is as defined in Claim 1.

4. The compound of any of Claims 1 to 3, wherein A is O and $R^1$ is H.

5. The compound of Claim 4, wherein R is H.

6. The compound of Claim 5 which has the formula

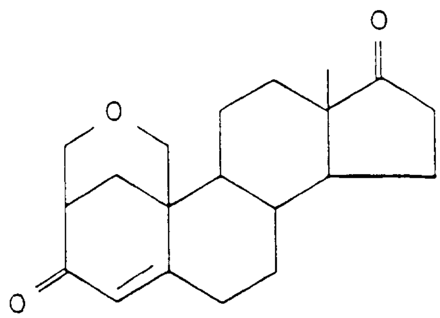

and is [3R-(3α,6aα,6bα,8aβ, 11aα,11bβ)] -3,4,6b,7,8,8a,10,11,11a,11b,12,13-dodecahydro-8a-methyl-6H-3,6a-methanocyclopenta-[5,6]napth[1,2-c]oxocin-2,9-dione.

7. The compound of Claim 3 which has the formula

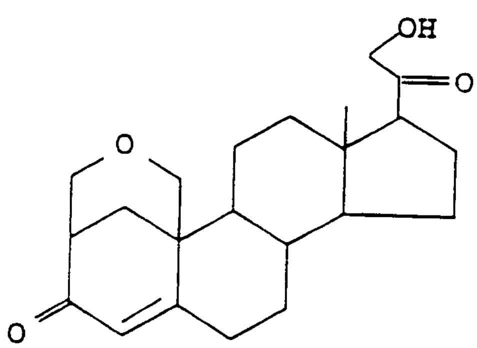

and is [3R-(3α,6aα,6bα,8aβ,9β,11aα,11bβ)] 3,4,7,8,8a,9,10,11a,11b,12,13-dodecahydro-9-(hydroxyacetyl)-8a-methyl-6H-3,6a-methanocyclopenta[5,6]napth[1,2-c]oxocin-2(6bH)-one.

8. A process for preparing a compound of the formula (I) as defined in any of Claims 1 to 7 which comprises

a) reacting a compound of the formula

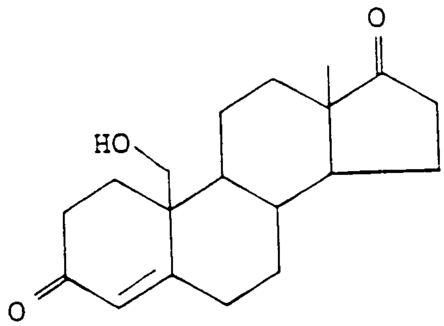

with diisopropylamine and 1-chloro-2,5-dioxahexane,

b) reacting the mixture with trimethylsilylchloride and lithium diisopropylamide, and

c) then reacting the mixture with $TiCl_4$ to yield the compound of Claims 1 to 7.

**9.** Use of a compound of the formula (I) according to any of Claims 1 to 7 for the preparation of a pharmaceutical composition.

**10.** Use according to Claim 9, wherein the composition is useful for inhibiting aromatase activity.

**11.** Use according to Claim 9, wherein the composition is useful for treating hyperestrogenemia.

**12.** Use according to Claim 9, wherein the composition is useful for treating estrogen-induced or estrogen-stimulated disorders.

**13.** Use according to Claim 9, wherein the composition is useful for producing an anti-fertility effect.

**14.** Use according to Claim 9, wherein the composition is useful for treating hypertensive or edemous conditions.

**15.** Use according to claim 9, wherein the composition is useful for treating breast cancer.

**16.** A pharmaceutical composition containing a compound of the formula (I) according to any of Claims 1 to 7 and optionally a pharmaceutically acceptable carrier and/or diluent.

**17.** The composition of Claim 16 for inhibiting aromatase activity.

**18.** The composition of Claim 16 for treating hyperestrogenemia.

**19.** The composition of claim 16 for treating estrogen-induced or estrogen-stimulated disorders.

**20.** The composition of claim 16 for producing an anti-fertility effect.

**21.** The composition of Claim 16 for treating hypertensive or edemous conditions.

**22.** The composition of claim 16 for treating breast cancer.

**Claims for the following Contracting States : GR, ES**

1. A compound of the formula (I)

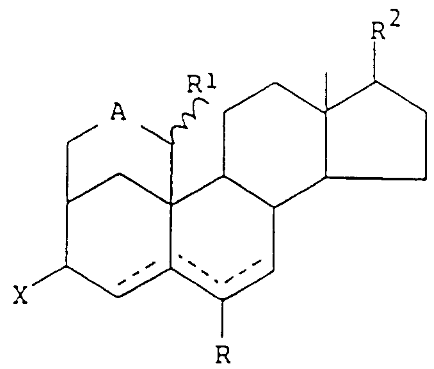

(I)

wherein
------- represents a single or double bond,
A is 0, S, SO, or $SO_2$,
R is H, $=CH_2$, $=O$, or -OH,
$R^1$ is H or $C_{1-4}$ alkyl,
$R^2$ is $=O$, -OH, -O-($C_{1-4}$ alkanoyl), or -CO-$CH_2$Y,
X is $=O$, $=CH_2$, -OH, or -O-($C_{1-4}$ alkanoyl), and
Y is H, -OH, or -O-($C_{1-4}$ alkanoyl), and when $R^2$ is -CO-$CH_2$Y, X may not include -OH, and R may not include $=O$ or -OH.

2. The compound (I) of Claim 1, wherein $R^2$ is $=O$, -OH, or -O-($C_{1-4}$ alkanoyl).

3. The compound of Claim 1, wherein $R^2$ is -CO-$CH_2$Y and Y is as defined in Claim 1.

4. The compound of any of Claims 1 to 3, wherein A is O and $R^1$ is H.

5. The compound of claim 4, wherein R is H.

6. The compound of Claim 5 which has the formula

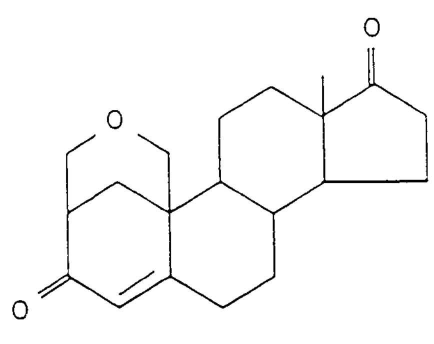

and is [3R-(3α,6aα,6bα,8aβ, 11aα,11bβ)] -3,4,6b,7,8,8a,10,11,11a,11b,12,13-dodecahydro-8a-methyl-6H-3,6a-methanocyclopenta-[5,6]napth[1,2-c]oxocin-2,9-dione.

7. The compound of Claim 3 which has the formula

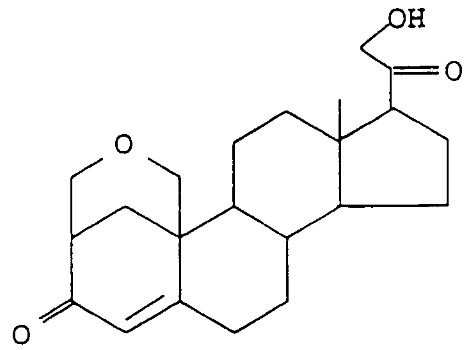

and is [3R-3α,6aα,6bα,8aβ,9β,11aα,11bβ)] 3,4,7,8,8a,9,10,11a,11b,12,13-dodecahydro-9-(hydroxyacetyl)-8a-methyl-6H-3,6a-methanocyclopenta[5,6]napth[1,2-c]oxocin-2(6bH)-one.

8. A process for preparing a compound of the formula (I) as defined in any of Claims 1 to 7 which comprises
   a) reacting a compound of the formula

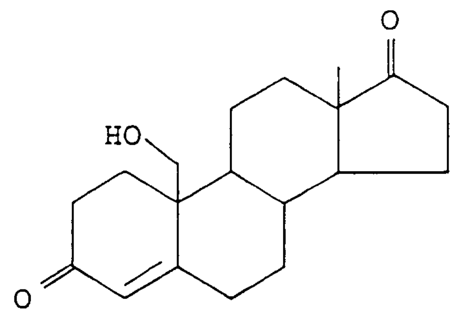

   with diisopropylamine and 1-chloro-2,5-dioxahexane,
   b) reacting the mixture with trimethylsilylchloride and lithium diisopropylamide, and
   c) then reacting the mixture with $TiCl_4$ to yield the compound of Claims 1 to 7.

9. A method for the preparation of a pharmaceutical composition comprising combining a compound of the formula (I) according to any of Claims 1 to 7 with a pharmaceutically acceptable carrier.

10. The method according to Claim 9, wherein the composition is useful for inhibiting aromatase activity.

11. The method according to Claim 9, wherein the composition is useful for treating hyperestrogenemia.

12. The method according to Claim 9, wherein the composition is useful for treating estrogen-induced or estrogen-stimulated disorders.

13. The method according to Claim 9, wherein the composition is useful for producing an anti-fertility effect.

14. The method according to Claim 9, wherein the composition is useful for treating hypertensive or edemous conditions.

15. The method according to claim 9, wherein the composition is useful for treating breast cancer.

**Patentansprüche**

**Patentansprüche für folgende Vertragsstaaten : AT, BE, CH, DE, DK, FR, GB, IT, LI, LU, NL, SE**

1. Verbindung der Formel (I)

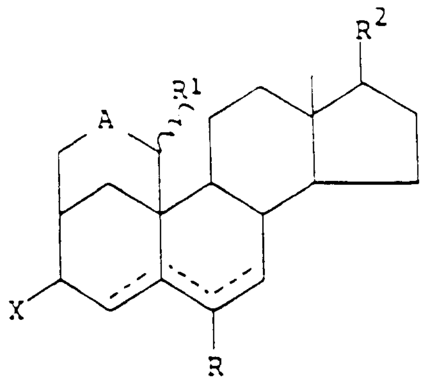

(I)

in der

------ eine Einfach- oder Doppelbindung bedeutet,

A ein Sauerstoff-, Schwefelatom, eine SO- oder $SO_2$-Gruppe ist,

R ein Wasserstoffatom, eine Gruppe der Formel $=CH_2$, $=O$ oder -OH ist,

$R^1$ ein Wasserstoffatom oder ein $C_{1-4}$-Alkylrest ist,

$R^2$ eine Gruppe der Formel $=O$, -OH oder ein -O-($C_{1-4}$-Alkanoyl)rest oder ein Rest der Formel $-CO-CH_2-Y$ ist,

X eine Gruppe der Formel $=O$, $=CH_2$, -OH oder ein -O-($C_{1-4}$-Alkanoyl)rest ist, und

Y ein Wasserstoffatom, eine Gruppe der Formel -OH oder ein -O-($C_{1-4}$-Alkanoyl)rest ist, und wenn $R^2$ ein Rest der Formel $-CO-CH_2-Y$ ist, X keine Gruppe der Formel -OH umfassen kann, und R keine Gruppe der Formel $=O$ oder -OH umfassen kann.

2. Verbindung (I) nach Anspruch 1, wobei $R^2$ eine Gruppe der Formel $=O$, -OH oder ein -O-($C_{1-4}$-Alkanoyl)rest ist.

3. Verbindung nach Anspruch 1, wobei $R^2$ ein Rest der Formel $-CO-CH_2-Y$ ist, und Y wie in Anspruch 1 definiert ist.

4. Verbindung nach einem der Ansprüche 1 bis 3, wobei A ein Sauerstoffatom ist, und $R^1$ ein Wasserstoffatom ist.

5. Verbindung nach Anspruch 4, wobei R ein Wasserstoffatom ist.

6. Verbindung nach Anspruch 5 der Formel

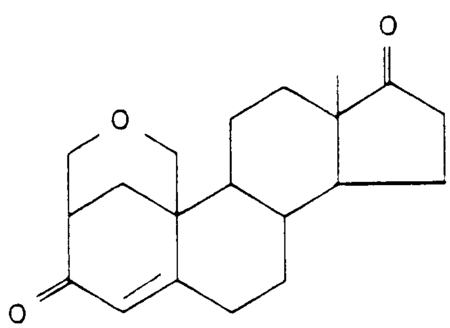

die [3R-(3α,6aα,6bα,8aβ,11aα,11bβ)]-3,4,6b,7,8,8a,10,11,11a,11b,12,-13-Dodecahydro-8a-methyl-6H-3,6a-methanocyclopenta[5,6]napth[1,2-c]oxocin-2,9-dion ist.

**7.** Verbindung nach Anspruch 3 der Formel

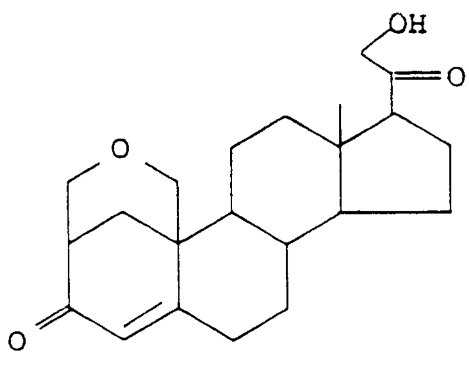

die [3R-(3α,6aα,6bα,8aβ,9β,11aα,11bβ)]-3,4,7,8,8a,9,10,11a,11b,12,13-Dodecahydro-9-(hydroxyacetyl)-8a-methyl-6H-3,6a-methanocyclopenta[5,6]napth[1,2-c]oxocin-2(6bH)-on ist.

**8.** Verfahren zur Herstellung einer Verbindung der Formel (I) nach einem der Ansprüche 1 bis 7, das umfaßt

a) die Umsetzung einer Verbindung der Formel

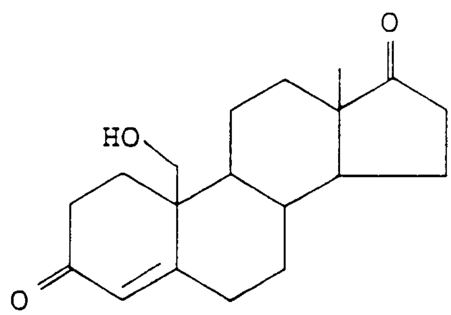

mit Diisopropylamin und 1-Chlor-2,5-dioxahexan,

b) die Umsetzung des Gemisches mit Trimethylsilylchlorid und Lithiumdiisopropylamid, und

c) anschließend die Umsetzung des Gemisches mit $TiCl_4$, wobei die Verbindung der Ansprüche 1 bis 7 entsteht.

**9.** Verwendung einer Verbindung der Formel (I) nach einem der Ansprüche 1 bis 7 zur Herstellung eines Arzneimittels.

**10.** Verwendung nach Anspruch 9, wobei das Mittel zur Inhibierung der Aromataseaktivität verwendbar ist.

**11.** Verwendung nach Anspruch 9, wobei das Mittel zur Behandlung von Hyperöstrogenämie verwendbar ist.

**12.** Verwendung nach Anspruch 9, wobei das Mittel zur Behandlung Östrogen-induzierter oder Östrogen-stimulierter Erkrankungen verwendbar ist.

**13.** Verwendung nach Anspruch 9, wobei das Mittel zur Erzeugung einer Antifertilitätswirkung verwendbar ist.

**14.** Verwendung nach Anspruch 9, wobei das Mittel zur Behandlung hypertonischer oder ödematöser Erkrankungen verwendbar ist.

**15.** Verwendung nach Anspruch 9, wobei das Mittel zur Behandlung von Brustkrebs verwendbar ist.

**16.** Arzneimittel mit einer Verbindung der Formel (I) nach einem der Ansprüche 1 bis 7 und gegebenenfalls einem pharmazeutisch verträglichen Träger und/oder Verdünnungsmittel.

**17.** Mittel nach Anspruch 16 zur Inhibierung der Aromataseaktivität.

**18.** Mittel nach Anspruch 16 zur Behandlung von Hyperöstrogenämie.

**19.** Mittel nach Anspruch 16 zur Behandlung Östrogen-induzierter oder Östrogen-stimulierter Erkrankungen.

**20.** Mittel nach Anspruch 16 zur Erzeugung einer Antifertilitätswirkung.

**21.** Mittel nach Anspruch 16 zur Behandlung hypertonischer oder ödematöser Erkrankungen.

**22.** Mittel nach Anspruch 16 zur Behandlung von Brustkrebs.

**Patentansprüche für folgende Vertragsstaaten : GR, ES**

**1.** Verbindung der Formel (I)

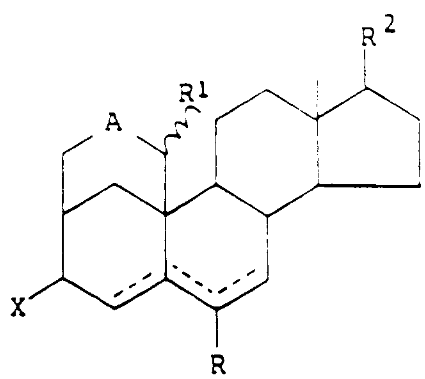

(I)

in der

------ eine Einfach- oder Doppelbindung bedeutet,

A ein Sauerstoff-, Schwefelatom, eine SO- oder $SO_2$-Gruppe ist,

R ein Wasserstoffatom, eine Gruppe der Formel $=CH_2$, $=O$ oder -OH ist,

$R^1$ ein Wasserstoffatom oder ein $C_{1-4}$-Alkylrest ist,

$R^2$ eine Gruppe der Formel $=O$, -OH oder ein -O-($C_{1-4}$-Alkanoyl)rest oder ein Rest der Formel -CO-$CH_2$-Y ist,

X eine Gruppe der Formel $=O$, $=CH_2$, -OH oder ein -O-($C_{1-4}$-Alkanoyl)rest ist, und

Y ein Wasserstoffatom, eine Gruppe der Formel -OH oder ein -O-($C_{1-4}$-Alkanoyl)rest ist, und wenn $R^2$ ein Rest der Formel -CO-$CH_2$-Y ist, X keine Gruppe der Formel -OH umfassen kann, und R keine Gruppe der Formel $=O$ oder -OH umfassen kann.

**2.** Verbindung (I) nach Anspruch 1, wobei $R^2$ eine Gruppe der Formel $=O$, -OH oder ein -O-($C_{1-4}$-Alkanoyl)rest ist.

**3.** Verbindung nach Anspruch 1, wobei $R^2$ ein Rest der Formel -CO-$CH_2$-Y ist, und Y wie in Anspruch 1 definiert ist.

4. Verbindung nach einem der Ansprüche 1 bis 3, wobei A ein Sauerstoffatom ist, und R[1] ein Wasserstoffatom ist.

5. Verbindung nach Anspruch 4, wobei R ein Wasserstoffatom ist.

6. Verbindung nach Anspruch 5 der Formel

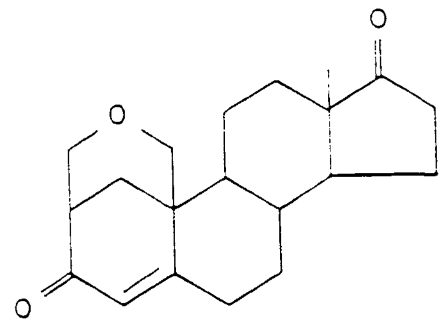

die [3R-(3α,6aα,6bα,8aβ,11aα,11bβ)]-3,4,6b,7,8,8a,10,11,11a,11b,12,-13-Dodecahydro-8a-methyl-6H-3,6a-methanocyclopenta[5,6]napth-[1,2-c]oxocin-2,9-dion ist.

7. Verbindung nach Anspruch 3 der Formel

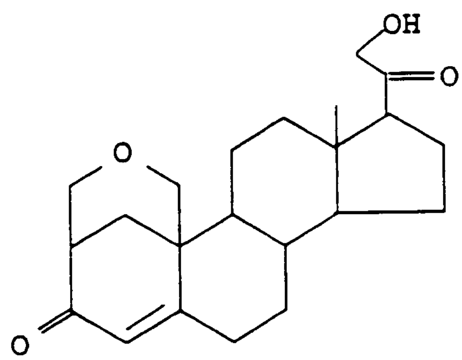

die [3R-(3α,6aα,6bα,8aβ,9β,11aα,11bβ)]-3,4,7,8,8a,9,10,11a,11b,12,13-Dodecahydro-9-(hydroxyacetyl)-8a-methyl-6H-3,6a-methanocyclopenta[5,6]napth[1,2-c]oxocin-2(6bH)-on ist.

8. Verfahren zur Herstellung einer Verbindung der Formel (I) nach einem der Ansprüche 1 bis 7, das umfaßt

a) die Umsetzung einer Verbindung der Formel

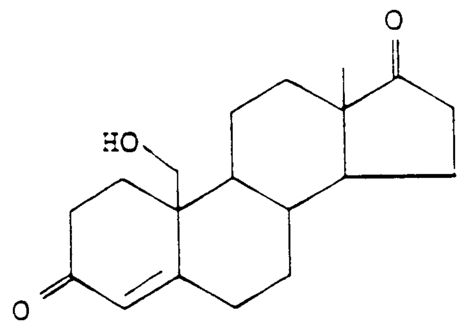

mit Diisopropylamin und 1-Chlor-2,5-dioxahexan,

b) die Umsetzung des Gemisches mit Trimethylsilylchlorid und Lithiumdiisopropylamid, und

c) anschließend die Umsetzung des Gemisches mit $TiCl_4$, wobei die Verbindung der Ansprüche 1 bis 7 entsteht.

**9.** Verfahren zur Herstellung eines Arzneimittels, umfassend die Kombination einer Verbindung der Formel (I) nach einem der Ansprüche 1 bis 7 mit einem pharmazeutisch verträglichen Träger.

**10.** Verfahren nach Anspruch 9, wobei das Mittel zur Inhibierung der Aromataseaktivität verwendbar ist.

**11.** Verfahren nach Anspruch 9, wobei das Mittel zur Behandlung von Hyperöstrogenämie verwendbar ist.

**12.** Verfahren nach Anspruch 9, wobei das Mittel zur Behandlung Östrogen-induzierter oder Östrogen-stimulierter Erkrankungen verwendbar ist.

**13.** Verfahren nach Anspruch 9, wobei das Mittel zur Erzeugung einer Antifertilitätswirkung verwendbar ist.

**14.** Verfahren nach Anspruch 9, wobei das Mittel zur Behandlung hypertonischer oder ödematöser Erkrankungen verwendbar ist.

**15.** Verfahren nach Anspruch 9, wobei das Mittel zur Behandlung von Brustkrebs verwendbar ist.

**Revendications**
**Revendications pour les Etats contractants suivants : AT, BE, CH, DE, DK, FR, GB, IT, LI, LU, NL, SE**

**1.** Un composé de formule (I) :

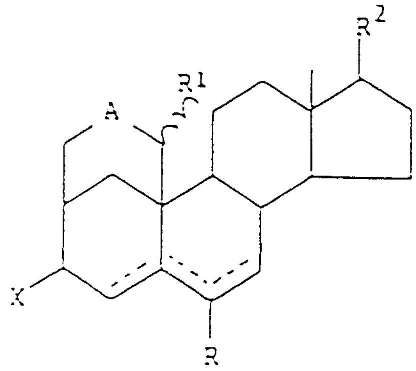

(I)

dans laquelle

-------- représente une liaison simple ou double,
A est O, S, SO ou $SO_2$,
R est H, $=CH_2$, =O, ou -OH,
$R^1$ est H ou $C_{1-4}$-alkyle,
$R^2$ est =O, -OH, ou -O-($C_{1-4}$-alcanoyle), ou -CO-$CH_2$-Y,
X est =O, $=CH_2$, -OH, ou -O-($C_{1-4}$-alcanoyle), et
Y est H, -OH ou -O-($C_{1-4}$-alcanoyle) et quand $R^2$ est -CO-$CH_2$-Y, X peut ne pas inclure-OH, et R peut ne pas inclure =O ou -OH.

**2.** Le composé (I) selon la revendication 1, selon laquelle $R^2$ est =O, -OH ou -O-($C_{1-4}$-alcanoyle).

**3.** Le composé selon la revendication 1, selon laquelle $R^2$ est -CO-$CH_2$-Y et Y est comme défini dans la revendication 1.

**4.** Le composé selon l'une quelconque des revendications 1 à 3, selon laquelle A est O et $R^1$ est H.

**5.** Le composé selon la revendication 4, selon laquelle R est H.

**6.** Le composé selon la revendication 5 qui a la formule

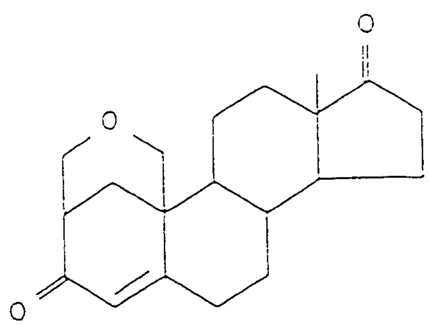

et qui est la [3R-(3α,6aα,6bα,8aβ,11aα,11bβ)]-3,4,6b,7,8,8a,10,11,11a,11b,12,-13-dodécahydro-8a-méthyl-6H-3,6a-méthanocyclopenta[5,6]-napht[1,2-c]oxocine-2,9-dione.

**7.** Le composé selon la revendication 3 qui a la formule

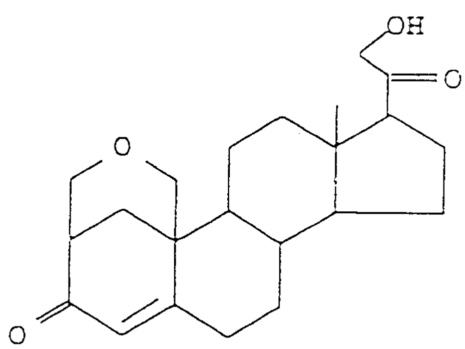

et qui est la [3R-(3α,6aα,6bα,8aβ,9β,11aα,11bβ)]-3,4,7,8,8a,9,10,11a,11b,12,13-dodécahydro-9-(hydroxyacétyl)-8a-méthyl-6H-3,6a-méthanocyclopenta[5,6]-napht[1,2-c]oxocine-2(6bH)-one.

**8.** Un procédé pour préparer un composé de formule (I) tel que défini dans l'une quelconque des revendications 1 à 7 qui comprend :

a) la réaction d'un composé de formule

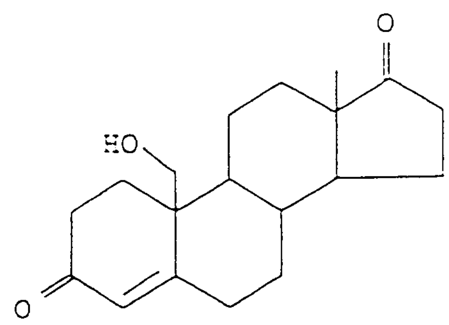

avec la diisopropylamine et le 1-chloro-2,5-dioxahexane
b) la réaction du mélange avec le chlorure de triméthylsilyle et le diisopropylamidure de lithium, et
c) puis la réaction du mélange avec le $TiCl_4$ pour donner le composé des revendications 1 à 7.

**9.** Utilisation d'un composé de formule (I) selon l'une quelconque des revendications 1 à 7 pour la préparation d'une composition pharmaceutique.

**10.** Utilisation selon la revendication 9, selon laquelle la composition est utile pour inhiber l'activité de l'aromatase.

**11.** Utilisation selon la revendication 9, selon laquelle la composition est utile pour le traitement de l'hyperoestrogénémie.

**12.** Utilisation selon la revendication 9, selon laquelle la composition est utile pour le traitement des désordres induits par un oestrogène ou stimulés par un oestrogène.

**13.** Utilisation selon la revendication 9, selon laquelle la composition est utile pour la production d'un effet antifécondité.

**14.** Utilisation selon la revendication 9, selon laquelle la composition est utile pour le traitement des états hypertensifs ou oedémateux.

**15.** Utilisation selon la revendication 9, selon laquelle la composition est utile pour le traitement du cancer du sein.

**16.** Une composition pharmaceutique contenant un composé de formule (I) selon l'une quelconque des revendications 1 à 7 et éventuellement un support et/ou un diluant pharmaceutiquement acceptable.

**17.** La composition selon la revendication 16 pour inhiber l'activité de l'aromatase.

**18.** La composition selon la revendication 16 pour le traitement de l'hyperoestrogénémie.

**19.** La composition selon la revendication 16 pour le traitement des désordres induits par un oestrogène ou stimulés par un oestrogène.

**20.** La composition selon la revendication 16 pour la production d'un effet antifécondité.

**21.** La composition selon la revendication 16 pour le traitement des états hypertensifs ou oedémateux.

**22.** La composition selon la revendication 16 pour le traitement du cancer du sein.

**Revendications pour les Etats contractants suivants : GR, ES**

**1.** Un composé de formule (I) :

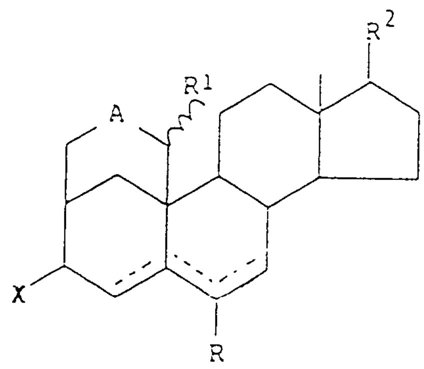

(I)

dans laquelle
-------- représente une liaison simple ou double,
A est O, S, SO ou $SO_2$,
R est H, $=CH_2$, =O, ou -OH,
$R^1$ est H ou $C_{1-4}$-alkyle,
$R^2$ est =O, -OH, ou -O-($C_{1-4}$-alcanoyle), ou -CO-$CH_2$-Y,
X est =O, $=CH_2$, -OH, -O-($C_{1-4}$-alcanoyle), et
Y est H, -OH ou -O-($C_{1-4}$-alcanoyle) et quand $R^2$ est -CO-$CH_2$ -Y, X peut ne pas inclure-OH, et R peut ne pas inclure =O ou -OH.

**2.** Le composé (I) selon la revendication 1, selon laquelle $R^2$ est =O, -OH ou -O-($C_{1-4}$-alcanoyle).

**3.** Le composé selon la revendication 1, selon laquelle $R^2$ est -CO-$CH_2$-Y et Y est comme défini dans la revendication 1.

**4.** Le composé selon l'une quelconque des revendications 1 à 3, selon laquelle A est O et $R^1$ est H.

**5.** Le composé selon la revendication 4, selon laquelle R est H.

**6.** Le composé selon la revendication 5 qui a la formule

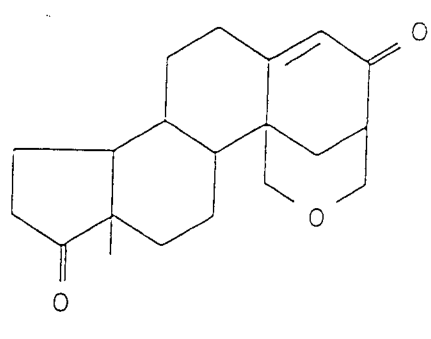

et qui est la [3R-(3α,6aα,6bα,8aβ,11aα,11bβ]-3,4,6b,7,8,8a,10,11,11a,11b,12,-13-dodécahydro-8a-méthyl-6H-3,6a-méthanocyclopenta[5,6]napht[1,2-c]oxocine-2,9-dione.

**7.** Le composé selon la revendication 3 qui a la formule

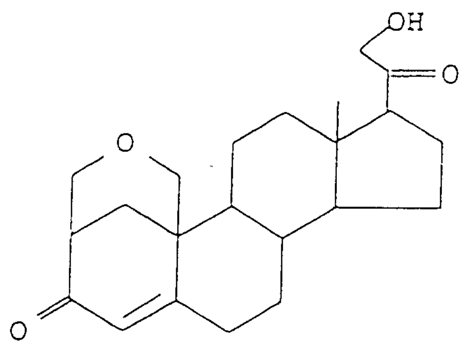

et qui est la [3R-(3α,6aα,6bα,8aβ,9β,11aα,11bβ]-3,4,7,8,8a,9,10,11a,11b,12,13-dodécahydro-9-(hydroxyacétyl)-8a-méthyl-6H-3,6a-méthanocyclopenta[5,6]-napht[1,2-c]oxocine-2(6bH)-one.

**8.** Un procédé pour préparer un composé de formule (I) tel que défini dans l'une quelconque des revendications 1 à 7 qui comprend :
a) la réaction d'un composé de formule

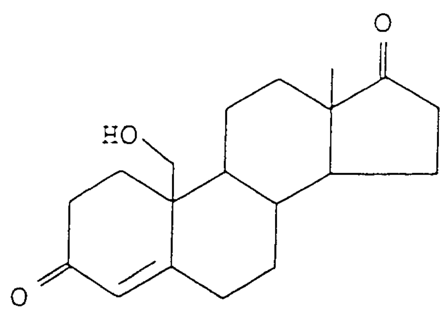

avec la diisopropylamine et le 1-chloro-2,5-dioxahexane
b) la réaction du mélange avec le chlorure de triméthylsilyle et le diisopropylamidure de lithium, et
c) puis la réaction du mélange avec le $TiCl_4$ pour donner le composé des revendications 1 à 7.

**9.** Une méthode pour la préparation d'une composition pharmaceutique comprenant la combinaison d'un composé de formule (I) selon l'une quelconque des revendications 1 à 7 avec un support pharmaceutiquement acceptable.

**10.** La méthode selon la revendication 9, selon laquelle la composition est utile pour inhiber l'activité de l'aromatase.

**11.** La méthode selon la revendication 9, selon laquelle la composition est utile pour le traitement de l'hyperoestrogénémie.

**12.** La méthode selon la revendication 9, selon laquelle la composition est utile pour le traitement des désordres induits par un oestrogène ou stimulés par un oestrogène.

**13.** La méthode selon la revendication 9, selon laquelle la composition est utile pour le production d'un effet antifécondité.

**14.** La méthode selon la revendication 9, selon laquelle la composition est utile pour le traitement des états hypertensifs ou oedémateux.

**15.** La méthode selon la revendication 9, selon laquelle la composition est utile pour le traitement du cancer du sein.